(19) Europäisches Patentamt
European Patent Office
Office européen des brevets

(11) Veröffentlichungsnummer: 0 437 844 A1

(12) EUROPÄISCHE PATENTANMELDUNG

(21) Anmeldenummer: 90125637.0

(22) Anmeldetag: 28.12.90

(51) Int. Cl.5: C07C 41/22, C07C 43/12

(30) Priorität: 18.01.90 DE 4001328

(43) Veröffentlichungstag der Anmeldung: 24.07.91 Patentblatt 91/30

(84) Benannte Vertragsstaaten: BE DE DK ES FR GB IT NL

(71) Anmelder: HOECHST AKTIENGESELLSCHAFT
Postfach 80 03 20
W-6230 Frankfurt am Main 80(DE)

(72) Erfinder: Stapel, Ralf, Dr.
Frankfurter Strasse 95
W-6233 Kelkheim/Taunus(DE)

# (54) Verfahren zur Herstellung von perfluorierten Ethern.

(57) Die Erfindung betrifft ein Verfahren zur Herstellung von perfluorierten Ethern der Formel I

$$R_f[(OCF(R)-CF_2)_nOCF_2-R']_m \qquad (I)$$

wobei
- $R_f$ = Rest eines Perfluoralkans mit 1-9 C-Atomen mit m freien Valenzen
- R, R' = F oder $CF_3$, wobei R und R' gleich oder verschieden sein können
- m = 1 oder 2
- n = ganze Zahl von 0 bis 100

aus Verbindungen der Formel II

$$R_f[(OCF(R)-CF_2)_nOCHF-R']_m \qquad (II)$$

wobei $R_f$, R, R', m und n die obige Bedeutung haben, durch Umsetzung mit $F_2$ bei 50-250° C.

EP 0 437 844 A1

# VERFAHREN ZUR HERSTELLUNG VON PERFLUORIERTEN ETHERN

Die Erfindung betrifft ein Verfahren zur Herstellung von perfluorierten Ethern der Formel I

$$R_f[(OCF(R)-CF_2)_nOCF_2-R']_m \qquad (I)$$

wobei

| | |
|---|---|
| $R_f$ = | Rest eines Perfluoralkans mit 1-9 C-Atomen und m freien Valenzen |
| R,R' = | F oder $CF_3$, wobei R und R' gleich oder verschieden sein können |
| m = | 1 oder 2 |
| n = | ganze Zahl von 0 bis 100 |

aus Verbindungen der Formel II

$$R_f[(OCF(R)-CF_2)_nOCHF-R']_m \qquad (II)$$

wobei $R_f$, R, R', m und n die obige Bedeutung haben.

Perfluorierte Ether, und speziell Perfluorpolyether, weisen außerordentlich hohe chemische und thermische Beständigkeit auf. Wegen ihrer besonderen physikalischen Eigenschaften, wie breiter Flüssigkeitsbereich, günstiger Viskositätsindex, geringe Oberflächenspannung und gute dielektrische Eigenschaften, haben diese Verbindungen zahlreiche Anwendungen als Inertflüssigkeiten und Schmiermittel in Gegenwart sehr aggressiv wirkender Medien gefunden.

Aus US-PS 3 595 925 ist die Herstellung von Verbindungen der Formel (I) durch Umsetzung von Verbindungen der Formel (II) mit $SbF_5$ bekannt. Das teure Fluorierungsmittel $SbF_5$ muß jedoch im allgemeinen im Überschuß eingesetzt werden, der nicht zurückgewonnen werden kann, und außerdem ist die Aufarbeitung des entstehenden Produktgemischs kompliziert. Überdies greift überschüssiges $SbF_5$ als starke Lewis-Säure den gewünschten Perfluorpolyether (I) unter Kettenabbau an, was zu Ausbeuteverlusten führt.

Es wurde nun gefunden, daß man die Fluorierung viel günstiger mit elementarem Fluor statt mit $SbF_5$ durchführen kann. Die gewünschten Perfluorpolyether (I) sind gegen $F_2$ unter den angewandten Bedingungen völlig stabil, weswegen die Ausbeute praktisch quantitativ ist. Nicht umgesetztes Fluor kann in einfacher Weise in den Prozeß zurückgeführt werden. Das gewünschte Endprodukt (I) fällt ohne Aufarbeitung in reiner Form an.

Gegenstand der Erfindung ist ein Verfahren zur Herstellung von perfluorierten Ethern der Formel I

$$R_f[(OCF(R)-CF_2)_nOCF_2-R']_m \qquad (I)$$

wobei

| | |
|---|---|
| $R_f$ = | Rest eines Perfluoralkans mit 1-9 C-Atomen und m freien Valenzen |
| R,R' = | F oder $CF_3$, wobei R und R' gleich oder verschieden sein können |
| m = | 1 oder 2 |
| n = | ganze Zahl von 0 bis 100 |

aus Verbindungen der Formel II

$$R_f[(OCF(R)-CF_2)_nOCHF-R']_m \qquad (II)$$

wobei $R_f$, R, R', m und n die obige Bedeutung haben, dadurch gekennzeichnet, daß man die Verbindungen

der Formel (II) mit $F_2$ bei 50-250 °C umsetzt.

Vorzugsweise verwendet man perfluorierte Ether der Formel II, die eine der Merkmalskombinationen 1-6 haben:

1. $R_f$ = n-$C_3F_7$, R= $CF_3$, R'= $CF_3$, m= 1, n eine der Zahlen 1-100
2. $R_f$ = -$C_2F_4$-, R= $CF_3$, R'= $CF_3$, m= 2, n " " " 5-100
3. $R_f$ = -$C_4F_8$-, R= $CF_3$, R'= $CF_3$, m= 2, n " " " 3-100
4. $R_f$ = $C_2F_5$, R= $CF_3$, R'= $CF_3$, m= 1, n " " " 10- 90
5. $R_f$ = $CF_3$, R= F, R'= F, m= 1, n " " " 10-100
6. $R_f$ = $C_2F_5$, R= F, R'= F, m= 1, n " " " 10-100.

Besonders bevorzugt verwendet man perfluorierte Ether der Formel II mit der eben genannten Merkmalskombination 1.

Die Ausgangsverbindungen der Formel (II) sind beispielsweise erhältlich durch Hydrolyse und Decarboxylierung (US-PS 3 342 875) von Oligomergemischen cyclischer perfluorierter Ether, wie Hexafluorpropylenoxid, die ihrerseits gemäß US-PS 3 412 148, DE-OS 1 520 527 oder EP-PS 0 196 904 hergestellt werden können. Die Verbindungen der Formel (II) fallen dabei im allgemeinen als Gemische mit mehr oder weniger breiter Molmassenverteilung an.

Die Umsetzung kann in einer Apparatur gemäß DE-OS 24 51 493 kontinuierlich, oder in einem Blasensäulenreaktor kontinuierlich oder diskontinuierlich durchgeführt werden.

Eine besonders einfache Ausführungsform besteht darin, daß man ein Oligomer der Formel (II) in ein einseitig geschlossenes, senkrecht stehendes Metallrohr füllt und am unteren Ende des Rohres über eine Begasungseinrichtung (z.B. Fritte oder Begasungsring) elementares Fluor einleitet. Die Länge des Rohres sollte das 5-fache des Innendurchmessers vorzugsweise nicht unterschreiten.

Das Fluor kann mit Inertgasen verdünnt oder unverdünnt eingeleitet werden.

Die Verweilzeiten im Reaktor liegen bevorzugt zwischen einigen Minuten und einigen Stunden

Der Druck ist für die Reaktion nicht kritisch. Bevorzugt wird unter Normaldruck gearbeitet.

Als Material für das Reaktionsrohr kommen alle unter den Reaktionsbedingungen hinreichend gegen Fluor beständigen Werkstoffe in Frage. Bevorzugt werden Kupfer, Stahl, Nickel oder Legierungen aus diesen Metallen.

Die Ausbeute an perfluorierten Ethern der Formel (I) liegt im allgemeinen über 95 %. Verluste treten nur durch Austragung niedrigsiedender Oligomerer der Formel (II) durch den Gasstrom auf. Die Reaktion erfolgt ansonsten quantitativ. Ausgetragene Oligomere können leicht in nachgeschalteten Vorlagen aufgefangen werden.

Nach Beendigung der Fluor-Zudosierung wird die Reaktionsmischung von gelöstem $F_2$ und HF durch Einleiten von Inertgas befreit.

Die Produkte (I) fallen als wasserklare, farblose und je nach Molmasse mehr oder weniger viskose Flüssigkeiten an. Falls ein Gemisch von Verbindungen (II) eingesetzt wird, entsteht ein Gemisch von Produkten (I), das durch Destillation zu den gewünschten Viskositätsklassen fraktioniert werden kann.

Die erhaltenen Produkte werden in der Elektronik- und Vakuum- sowie der chemischen Industrie als funktionelle Flüssigkeiten und Schmiermittel eingesetzt.

**Beispiel 1**

In ein einseitig geschlossenes 10 cm weites und 1 m langes Ni-Rohr wurden 8.0 kg eines Gemisches von Oligomeren der Formel (II) mit $R_f$ = n-$C_3F_7$, R = $CF_3$, m = 1 und n etwa 8-30 (Mittelwert von n etwa 14) gefüllt. Am Boden des Reaktors war zur Gaseinleitung ein Begasungsring (8 cm Durchmesser, 36 Bohrungen mit 1 mm Durchmesser) angebracht. Mittels Außenheizung wurde das Oligomer auf 200 °C (Innentemperatur) erhitzt, wobei 20 $lh^{-1}$ $N_2$ eingeleitet wurden. Anschließend wurde ein Gemisch aus 10 $lh^{-1}$ $N_2$ und 10 $lh^{-1}$ $F_2$ 20 h lang zudosiert. Ansohließend wurde die $F_2$-Zufuhr gestoppt und bei der gleichen Temperatur mit $N_2$ entgast.

Man erhielt 7878 g perfluoriertes Öl, das -$OCF_2CF_3$-Endgruppen besitzt ($^{19}$F-NMR). Im $^1$H-NMR war kein Restprotonengehalt feststellbar.

**Beispiel 2**

In den Fluorierungsreaktor gemäß Beispiel 1 wurden 8.9 kg eines Gemischs von Oligomeren der Formel (II) mit $R_f$ = n-$C_3F_7$, R = $CF_3$, n etwa 30-60 (Mittelwert etwa 46) und m = 1 mit einem Siedebereich von 200-340 °C bei $10^{-3}$ mbar und der kinematischen Viskosität von 387 cSt bei 40 °C gegeben. Das Oligomer wurde 14 h mit einem Gemisch aus 10 $lh^{-1}$ $F_2$ und 10 $lh^{-1}$ $N_2$ bei einer Temperatur von 200 °C behandelt und anschließend mit $N_2$ entgast.

Nach Entleeren des Reaktors erhielt man 8492 g eines perfluorierten Öls der Formel (I) mit $R_f$ = n-$C_3F_7$, R = $CF_3$, m = 1, Mittelwert von n etwa 46 und einer kinematischen Viskosität von 382 cSt bei 40 °C.

**Patentansprüche**

**1.** Verfahren zur Herstellung von perfluorierten Ethern der Formel (I)

$$R_f[(O\underset{\displaystyle R}{\underset{|}{C}}F-CF_2)_nOCF_2-R']_m \qquad (I)$$

wobei

| | | |
|---|---|---|
| $R_f$ | = | Rest eines Perfluoralkans mit 1-9 C- Atomen und m freien Valenzen |
| R,R' | = | F oder $CF_3$, wobei R und R' gleich oder verschieden sein können |
| m | = | 1 oder 2 |
| n | = | ganze Zahl von 0 bis 100 |

aus Verbindungen der Formel II

$$R_f[(O\underset{\displaystyle R}{\underset{|}{C}}F-CF_2)_nOCHF-R']_m \qquad (II)$$

wobei $R_f$, R, R', m und n die obige Bedeutung haben, dadurch gekennzeichnet, daß man die Verbindungen der Formel (II) mit $F_2$ bei 50-250 °C umsetzt.

**2.** Verfahren nach Anspruch 1, dadurch gekennzeichnet, daß man Verbindungen der Formel II einsetzt, in der die Variablen folgende Werte haben
$R_f$ = n-$C_3F_7$, R = $CF_3$, R' = $CF_3$, m = 1, n eine der Zahlen 1-100

**3.** Verfahren nach Anspruch 1, dadurch gekennzeichnet, daß man Verbindungen der Formel II einsetzt, in der die Variablen folgende Werte haben
$R_f$ = -$C_2F_4$-, R = $CF_3$, R' = $CF_3$, m = 2, n eine der Zahlen 5-100

**4.** Verfahren nach Anspruch 1, dadurch gekennzeichnet, daß man Verbindungen der Formel II einsetzt, in der die Variablen folgende Werte haben
$R_f$ = -$C_4F_8$-, R = $CF_3$, R' = $CF_3$, m = 2, n eine der Zahlen 3-100

**5.** Verfahren nach Anspruch 1, dadurch gekennzeichnet, daß man Verbindungen der Formel II einsetzt, in der die Variablen folgende Werte haben
$R_f$ = $C_2F_5$ , R = $CF_3$, R' = $CF_3$, m = 1, n eine der Zahlen 10- 90

**6.** Verfahren nach Anspruch 1, dadurch gekennzeichnet, daß man Verbindungen der Formel II einsetzt, in der die Variablen folgende Werte haben
$R_f$ = $CF_3$ , R = F , R' = F , m = 1, n eine der Zahlen 10-100

**7.** Verfahren nach Anspruch 1, dadurch gekennzeichnet, daß man Verbindungen der Formel II einsetzt, in der die Variablen folgende Werte haben
$R_f$ = $C_2F_5$ , R = F , R' = F , m = 1, n eine der Zahlen 10-100.

Europäisches Patentamt

# EUROPÄISCHER RECHERCHENBERICHT

Nummer der Anmeldung

**EP 90 12 5637**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | Progress in inorganic chemistry vol. 26, 1979, Interscience Publ. New York, USA Seiten 162 - 210; R.J. Lagow, J.L. Margrave: "Direct Fluorination: A "New" Approach to Fluorine Chemistry "<br>* S. 167-8, 179 * | 1 | C 07 C 41/22<br>C 07 C 43/12 |
| A | Journal of Organic Chemistry vol. 53, no. 1, 1988, American Chemical Society, Ohio, USA Seiten 78 - 85; H-N. Huang: "Synthesis of unusual perfluorocarbon ethers and amines containing bulkyfluorocarbon groups: New biomedical materials."<br>* S. 79, Abb. 1 * | 1 | |
| A | EP-A-0 332 601 (MONSANTO)<br>* Anspruch 1 * | 1 | |
| A | EP-A-0 077 114 (GREEN CROSS CORP.)<br>* Seite 12, Zeile 23 - Seite 13, Zeile 16 * | 1 | |
| A | EP-A-0 269 029 (AUSIMONT)<br>* Anspruch 1 * | 1 | RECHERCHIERTE SACHGEBIETE (Int. Cl.5) |
| D,A | US-A-3 595 925 (B.H. GARTH)<br>* Spalte 1, Zeile 46 - Spalte 1, Zeile 58 * | 1 | C 07 C |

Der vorliegende Recherchenbericht wurde für alle Patentansprüche erstellt

| Recherchenort | Abschlußdatum der Recherche | Prüfer |
|---|---|---|
| Berlin | 10 April 91 | PROBERT C.L. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument